# DEMANDE DE BREVET EUROPEEN

(11) **EP 2 875 818 A1**
(43) Date de publication de la demande: **27.05.2015**
(21) Numéro de dépôt: 14306580.3
(22) Date de dépôt: 08.10.2014
(51) Int. Cl.: A61K 33/00, A61P 25/06

(54) **Traitement des migraines chroniques par inhalation d'oxygène**

(30) Priorité: 26.11.2013 FR 1361628
(71) Demandeur: Air Liquide Santé (International), 75007 Paris (FR)
(72) Inventeur: Ellenberg, Eytan, 75019 Paris (FR)
(74) Mandataire: Pittis, Olivier

(57) **Abrégé**

L'invention concerne une composition gazeuse inhalable contenant de l'oxygène (O₂) en une proportion supérieure à 50% en volume, destinée à traiter, à minimiser ou à prévenir une crise de migraine chronique chez un malade, ladite composition étant produite sur site, c'est-à-dire au domicile du malade, par un dispositif de production d'oxygène conçu pour produire un flux de gaz contenant plus de 50% d'oxygène à partir d'air.

## Description

L'invention concerne une composition gazeuse riche en oxygène produite sur site, c'est-à-dire au domicile du patient, et utilisée pour la gestion des crises de migraine chronique chez les patients à leur domicile.

La migraine chronique est une complication fréquente des migraines classiques, c'est-à-dire des céphalées ou maux de tête classiques.

Une migraine chronique se caractérise par la survenue de plus de 15 crises de céphalées par mois pendant au moins 3 mois avec au moins 8 jours de migraine au total, d'après la Société Française d'Etude des Migraines et des Céphalées.

Afin de tenter de se soulager de leurs migraines chroniques, certains malades souffrant de migraines chroniques ont tendance à augmenter la quantité et/ou les doses de médicaments qu'ils prennent, à savoir typiquement des comprimés de type anti-inflammatoires non stéroidiens (AINS) qui diminuent l'inflammation des vaisseaux méningés ou de type triptans qui sont des vasoconstricteurs réduisant le diamètre des vaisseaux sanguins dilatés.

Or, l'augmentation de la quantité et/ou des doses de médicaments absorbés engendre un risque élevé d'abus médicamenteux chez ces malades et des conséquences sur la santé de ces patients, notamment en termes d'effets secondaires, d'interactions médicamenteuses, d'accoutumance...

A l'inverse, certains malades sont effrayés par le risque d'abus médicamenteux et tentent de réduire les doses d'antimigraineux, ce qui se fait alors au dépend d'un soulagement de leur migraine chronique.

Par ailleurs, certains malades ne peuvent être soignés à l'aide de ces comprimés du fait de leurs effets secondaires. Ainsi, les médicaments de type AINS sont contre-indiqués chez les patients ayant des antécédents d'ulcère gastroduodénal et déconseillés chez ceux ayant des antécédents de maladies rénales. De façon analogue, les triptans sont contre-indiqués chez les patients ayant des antécédents cardio-vasculaires, tels qu'infarctus, hypertension artérielle, accident vasculaire cérébral...

En outre, l'impact social négatif des migraines chroniques est très important puisqu'il engendre notamment des arrêts de travail répétés, une solitude accrue des personnes qui ont tendance s'isoler...

Par ailleurs, le document FR-A-2929513 propose d'utiliser de l'oxygène gazeux pour traiter les migraines sans aura chez la femme enceinte. L'oxygène utilisé provient de bouteilles de gaz. Cette solution n'est pas pratique car elle requiert la mise en place d'une logistique de gestion des bouteilles de gaz et n'est donc pas aisément applicable au traitement des patients à leur domicile.

Le problème qui se pose alors est de proposer une composition efficace, sans effet secondaire et simple à administrer, qui permette de traiter, c'est-à-dire d'au moins diminuer, la douleur engendrée par les céphalées chez des malades souffrant de migraines chroniques, et qui par ailleurs permette aux malades de se soigner rapidement dès la survenue de leur crise et sans avoir besoin de se rendre dans un centre de soins, tel un médecin, un hôpital ou analogue.

La solution de l'invention concerne une composition gazeuse inhalable, c'est-à-dire un médicament gazeux inhalable, contenant de l'oxygène (O₂) en une proportion supérieure à 50% en volume, destinée à traiter, à minimiser ou à prévenir un état de migraine chronique chez un être humain, ladite composition gazeuse étant produite sur site, c'est-à-dire au domicile du patient, par un dispositif de production d'oxygène conçu pour produire un flux de gaz contenant plus de 50% en volume d'oxygène à partir d'air.

Dans le cadre de la présente invention, la gestion des crises de migraine se réalise donc directement au domicile qui représente le lieu idéal de soulagement des crises puisqu'il offre aux malades, un environnement connu, avec possibilité d'isolement et d'atténuation des sons, des lumières ou autres facteurs stressants ou perturbants.

Dans le cadre de la présente invention :
- la composition gazeuse permet préférentiellement de traiter ou minimiser une crise de migraine.
- les pourcentages (%) de gaz sont des pourcentages volumiques ;
- « la migraine chronique » est définie comme des crises de céphalée (maux de tête) ayant lieu plus de 15 fois par mois pendant au moins 3 mois avec au moins 8 jours de migraine au total ;
- « composition gazeuse produite sur site » signifie que la composition gazeuse est fabriquée directement sur le lieu de son utilisation, au domicile principalement, donc de son administration par inhalation au patient, et ce, par opposition à un médicament qui fabriqué en usine ou ailleurs, puis acheminé sur son site d'utilisation dans un conteneur, telle une bouteille de gaz par exemple.

Selon le cas, la composition gazeuse inhalable l'invention peut comprendre l'une ou plusieurs des caractéristiques techniques suivantes :
- elle contient une proportion de d'oxygène supérieure à 60%.
- elle contient une proportion d'oxygène supérieure à 70%.
- elle contient une proportion d'oxygène supérieure à 80%.
- elle contient typiquement une proportion d'oxygène de 85 à 96%.
- elle est obtenue par séparation d'air au moyen d'un dispositif de production d'oxygène choisi parmi les concentrateurs d'oxygène.
- elle est obtenue par séparation d'air au moyen d'au moins un matériau adsorbant agencé dans le dispositif de production d'oxygène.
- elle est obtenue par adsorption préférentielle de l'azote sur au moins un matériau adsorbant et récupération d'un flux de gaz enrichi en oxygène.
- au moins un matériau adsorbant comprend une zéolite, de préférence une zéolite au moins en partie échangée par des cations lithium, calcium.....
- le matériau adsorbant comprend avantageusement une zéolite échangée à au moins 80% par des cations lithium (Li), de préférence entre 88 et 98% par des cations lithium, les cations résiduels étant essentiellement du sodium (Na).
- elle est obtenue par adsorption selon des cycles de production de type PSA (Pressure Swing Adsorption = adsorption à pression modulée), VSA (Vacuum Swing Adsorption = adsorption à vide modulé), ou une combinaison de tels cycles.
- elle est administrée pendant la crise de migraine.
- elle est administrée le plus rapidement possible après le début de crise.
- l'administration de la composition gazeuse est poursuivie jusqu'à disparition totale ou partielle des symptômes de migraine.
- la durée d'inhalation est choisie pour conduire à au moins une diminution de la douleur de migraine.
- la durée d'inhalation est choisie pour conduire à une disparition complète de la douleur de migraine.
- le temps d'inhalation de la composition gazeuse est d'au moins 10 minutes, de préférence de l'ordre de 15 minutes à 3 heures.
- l'administration de la composition gazeuse se fait de manière continue ou discontinue, de préférence de manière continue.
- l'administration de la composition gazeuse se fait de manière continue à un débit de 1 l/min à 30 l/min, de préférence entre 3 et 15 1/min, de préférence encore de 5 à 12 1/min, typiquement de l'ordre de 6 à 10 l/min.
- l'administration de la composition gazeuse se fait au moyen d'une interface patient, de préférence une interface patient choisie parmi les masques respiratoires et les canules nasales.
- l'administration de la composition gazeuse se fait au moyen d'un masque respiratoire dit « à haute concentration » comprenant un corps de masque comportant une chambre d'inhalation en communication fluidique avec un réservoir souple contenant au moins une partie de la composition gazeuse.
- l'administration de la composition gazeuse se fait au moyen d'un masque respiratoire comportant une valve anti-retour agencée entre la chambre d'inhalation et le réservoir souple.
- l'être humain est un homme ou une femme, y compris les enfants.
- la composition gazeuse est fabriquée au domicile du patient à partir d'air ambiant.
- la composition gazeuse est fabriquée au domicile du patient et immédiatement avant ou au moment de son inhalation par ledit patient.

Par ailleurs, l'invention porte aussi sur un procédé de fabrication d'une composition gazeuse inhalable, c'est-à-dire d'un médicament inhalable, contenant de l'oxygène en une proportion supérieure à 50% en volume, destinée à traiter, à minimiser ou à prévenir un état de migraine chronique chez un être humain, dans lequel ladite composition gazeuse est produite sur site, c'est-à-dire au domicile du patient, par un dispositif de production d'oxygène conçu pour produire un flux de gaz contenant plus de 50% en volume d'oxygène à partir d'air, en particulier un concentrateur d'oxygène tel que décrit ci-avant.

En outre, l'invention porte également sur une méthode de traitement thérapeutique permettant de traiter, minimiser ou prévenir un état de migraine chronique chez un individu, à savoir un homme ou une femme souffrant de migraines chroniques, dans laquelle :
i) on produit sur site, c'est-à-dire directement sur le lieu d'utilisation, typiquement au domicile du patient, une composition gazeuse, c'est-à-dire un médicament gazeux, inhalable contenant plus de 50% d'oxygène, typiquement plus de 80% d'oxygène, au moyen d'un dispositif de production d'oxygène conçu pour produire un flux de gaz contenant plus de 50% d'oxygène à partir d'air ; et
ii) on administre par inhalation audit individu ladite composition gazeuse contenant de l'oxygène en une proportion supérieure à 50% en volume obtenu à l'étape i).

Selon le cas, la méthode de l'invention peut comprendre l'une ou plusieurs des caractéristiques techniques suivantes:
- le dispositif de production d'oxygène est un concentrateur d'oxygène, notamment tel que décrit ci-avant.
- le dispositif de production d'oxygène produit et délivre un flux continu ou quasicontinu de ladite composition gazeuse.
- l'administration de ladite composition gazeuse se fait par inhalation continue du médicament gazeux.
- l'administration de ladite composition gazeuse est opérée au moment des crises de migraine, de préférence jusqu'à disparition de la douleur migraineuse.
- l'administration de ladite composition gazeuse est opérée pendant une durée de 15 min à 1 h 30 min, typiquement pendant moins de 1 h.
- l'administration de ladite composition gazeuse est opérée à un débit de 3 à 15 l/min.
- ladite composition gazeuse contient de 85 à 96% d'oxygène.

D'une façon générale, le fait que ladite composition gazeuse, i.e. médicament gazeux, riche en oxygène soit, dans le cadre de la présente invention, produite directement sur site (in situ) par séparation d'air ambiant au moyen d'un dispositif de production d'oxygène, tel un concentrateur d'oxygène, est particulièrement avantageux car :
- cela évite une logistique lourde de transport,
- cela minimise les ruptures d'approvisionnement et donc les risques pour le patient, notamment que ce dernier se retrouve à cours de gaz thérapeutique,
- permet de correspondre au mieux au lieu idéalement choisi par les patients en cas de crise sévère (domicile)
- il présente un coût moindre pour les autorités de santé, comparé à l'acheminement de bouteilles en quantités chez le patient ou son pharmacien

La présente invention va maintenant être décrite plus en détail en références aux Figures annexées parmi lesquelles :
- la Figure 1 représente une vue schématique d'un dispositif A de production d'oxygène conçu pour produire, in situ, une composition gazeuse selon la présente invention à partir d'air ambiant ; et
- la Figure 2 schématise (vue latérale) un masque dit « à haute concentration » utilisable pour administrer la composition gazeuse selon la présente invention.

Plus précisément, le dispositif 1 de production d'oxygène est un concentrateur d'oxygène qui permet de produire la composition gazeuse faisant office de médicament gazeux selon l'invention sur site, c'est-à-dire directement au domicile du patient, par séparation de l'air ambiant (teneur en O₂ environ 21 vol. %) par adsorption préférentielle de l'azote sur un (ou plusieurs) matériau(x) adsorbant(s) et récupération d'un flux de gaz enrichi en oxygène, c'est-à-dire contenant plus de 50% en volume d'oxygène, en particulier plus de 80 vol.% d'oxygène, typiquement de 85 à 96% d'oxygène, le reste étant essentiellement de l'azote et minoritairement d'autres impuretés du type argon.

Avantageusement, le matériau adsorbant est formé de particules ou billes de zéolite échangée par des cations métalliques. Préférentiellement, la zéolite est échangée à plus de 80% par des cations lithium. Typiquement, on utilise une zéolite échangée entre 88 et 98% avec des cations Li, les cations résiduels étant principalement du sodium et/ou éventuellement du potassium. Une telle zéolite échangée au lithium et le procédé de séparation d'air et de production d'oxygène la mettant en oeuvre sont décrits notamment par EP-A-0297542.

Les particules de matériau adsorbant sont agencées au sein d'un ou plusieurs lits, de préférence plusieurs lits, typiquement 2 ou 3 lits, agencés en parallèle et fonctionnant de façon alternée. L'adsorption de l'azote et la production du flux d'oxygène utilisé en tant que composition gazeuse inhalable selon l'invention se fait alors par cycles de type PSA ou VSA comprenant une ou des phases de production et/ou une ou des phases de régénération opérées à des pressions différentes.

Une fois produit au sein du concentrateur 1 d'oxygène, le flux gazeux constituant la composition gazeuse selon l'invention est acheminé par un conduit flexible 18 jusqu'à une interface patient 10, tel un masque respiratoire, qui permet de l'administrer par inhalation, continue ou fractionnée, à un être humain 2, qu'il soit un homme ou une femme, et quel que soit son âge, souffrant de migraines chroniques se caractérisant par des crises de céphalée (maux de tête) ayant lieu plus de 15 fois par mois pendant au moins 3 mois avec au moins 8 jours de migraine au total.

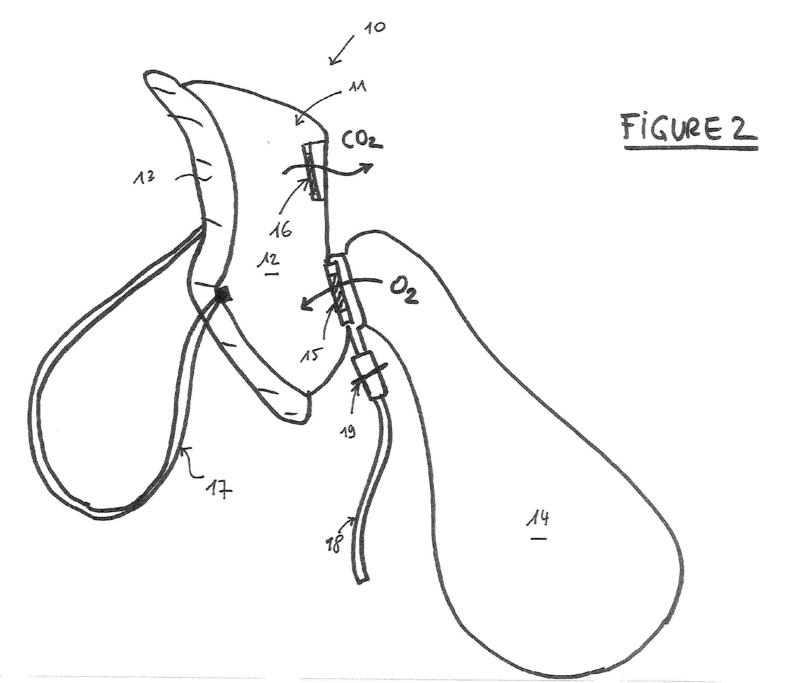

De façon avantageuse, dans le cadre de la présente invention, pour administrer le gaz au patient, on utilise un masque facial 10 dit « à haute concentration» . Un tel masque 10 est schématisé sur la Figure 2.

Ce masque respiratoire 10 comprend une coque 11 formant corps de masque. La coque 11 inclut une chambre d'inhalation 12 de gaz venant se positionner sur les régions nasale et buccale du patient, lorsque celui-ci porte le masque 10. L'étanchéité est assurée par une bordure formée d'un coussinet 13 flexible, par exemple en silicone venant épouser les contours des régions faciales du patient, notamment l'arête nasale, les régions des joues situées de part et d'autre du nez, et la région sous la bouche, c'est-à-dire au niveau du menton du patient.

La chambre d'inhalation 12 est en communication fluidique avec un réservoir souple de gaz 14 formé d'une poche flexible typiquement en polymère (volume environ 0,5 litre) qui est remplie avec de l'oxygène produit par le concentrateur d'oxygène.

Une valve anti-retour 15 agencée entre le réservoir souple 14 et la chambre d'inhalation 12 permet d'éviter au CO₂ expiré par le patient de se mélanger à l'oxygène contenu dans le réservoir souple 14. Le CO₂ expiré est évacué de la chambre d'inhalation 12 par une valve expiratoire 16 agencée dans le corps du masque 10.

Le maintien du masque 10 en position sur le visage du patient se fait au moyen d'une sangle flexible ou d'un élastique 17.

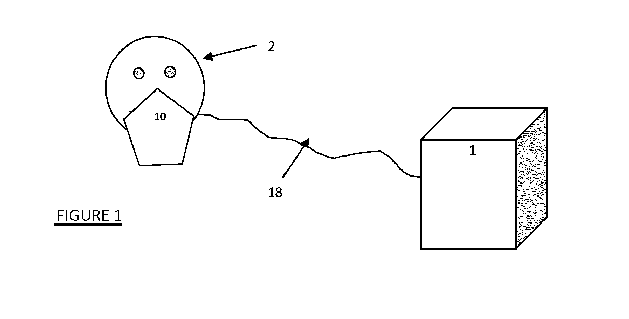

Le remplissage du réservoir souple 14 avec le flux d'oxygène produit par le concentrateur d'oxygène 1 (cf. Fig. 1) se fait via le tuyau flexible 18 venant se raccorder au masque 10 via un connecteur 19 adapté.

Ce type de masque facial 10 permet d'administrer de plus fortes concentrations d'oxygène aux patients que les masques respiratoires classiques, c'est-à-dire sans réservoir souple, ou des débits d'oxygène plus importants à concentration égale de sorte d'augmenter la FiO₂ du patient, dans le cadre d'un haut débit, i.e. 9 1/min par exemple.

La durée d'inhalation de la composition gazeuse est choisie, au cas par cas, pour conduire à une diminution et de préférence à une disparition totale de la douleur de migraine chez le patient considéré. Typiquement, elle est d'au moins 15 minutes à 3 heures. Le débit d'administration est quant à lui de l'ordre de 1 1/min à 30 1/min, typiquement de l'ordre de 9 à 11 l/min.

### Exemple

Afin de vérifier l'efficacité de la composition gazeuse, c'est-à-dire du médicament gazeux, selon l'invention, on a administré une composition gazeuse inhalable selon l'invention à un patient souffrant de migraines chroniques depuis des années, à savoir une femme de 40 ans.

La composition gazeuse administrée a consisté en un flux gazeux riche en oxygène produit directement au domicile du patient, c'est-à-dire in situ par un dispositif de production d'oxygène de type concentrateur d'oxygène fonctionnant par cycle de type PSA.

Dans le cas présent, on a mis en oeuvre le concentrateur d'oxygène de dénomination commerciale Platinum 9™ de la société Invacare™ qui alimentait en flux riche en oxygène un masque respiratoire à haute concentration de la société Intersurgical™, du type de celui représenté en Figure 2.

Ce concentrateur permet de produire un flux de gaz enrichi en oxygène à un débit allant de 3 à 9 1/min et à une concentration en oxygène de 87 à 93 vol.% d'oxygène, en fonction du débit choisi.

Dans le cadre du présent Exemple, l'administration du flux d'oxygène produit in situ se fait au moment des crises de migraine, pendant une durée de 30 minutes et à un débit de 9 l/min.

Les résultats obtenus conduisant à un soulagement des crises au bout de 8 à 10 minutes chez le patient ainsi traité.

Ceci démontre l'efficacité de la composition gazeuse selon l'invention dans le traitement des migraines chroniques lorsque ladite composition gazeuse est produite directement sur site, c'est-à-dire directement au domicile du patient et juste avant son inhalation.

## Revendications

1. Composition gazeuse inhalable contenant de l'oxygène (O₂) en une proportion supérieure à 50% en volume, destinée à traiter, à minimiser ou à prévenir une crise de migraine chronique chez un être humain, ladite composition étant produite sur site par un dispositif de production d'oxygène conçu pour produire un flux de gaz contenant plus de 50% d'oxygène à partir d'air.

2. Composition gazeuse selon la revendication précédente, **caractérisée en ce qu'**elle contient une proportion en volume d'oxygène supérieure à 60%.

3. Composition gazeuse selon l'une des revendications précédentes, **caractérisée en ce qu'**elle contient une proportion de d'oxygène supérieure à 70%.

4. Composition gazeuse selon l'une des revendications précédentes, **caractérisée en ce qu'**elle contient une proportion d'oxygène supérieure à 80%.

5. Composition gazeuse selon l'une des revendications précédentes, **caractérisée en ce qu'**elle contient une proportion d'oxygène inférieure à 100%.

6. Composition gazeuse selon l'une des revendications précédentes, **caractérisée en ce qu'**elle contient une proportion d'oxygène inférieure à 96%.

7. Composition gazeuse selon l'une des revendications précédentes, **caractérisée en ce qu'**elle est obtenue par séparation d'air au moyen d'au moins un matériau adsorbant agencé dans le dispositif concentrateur d'oxygène.

8. Composition gazeuse selon l'une des revendications précédentes, **caractérisée en ce qu'**au moins un matériau adsorbant est une zéolite, de préférence une zéolite au moins en partie échangée par des cations lithium et/ou calcium.

9. Composition gazeuse selon l'une des revendications précédentes, **caractérisée en ce qu'**elle est destinée à être administrée pendant une crise de migraine, de préférence le plus rapidement possible après le début de crise et l'administration est poursuivie jusqu'à disparition totale ou partielle des symptômes de migraine.

10. Composition gazeuse selon l'une des revendications précédentes, **caractérisée en ce que** l'être humain est un homme ou une femme.

11. Composition gazeuse selon l'une des revendications précédentes, **caractérisée en ce qu'**elle est fabriquée au domicile du patient à partir d'air ambiant.

12. Composition gazeuse selon l'une des revendications précédentes, **caractérisée en ce qu'**elle est administrée au moyen d'un masque respiratoire comprenant un corps de masque comprenant une chambre d'inhalation en communication fluidique avec un réservoir souple contenant au moins une partie de la composition gazeuse.

13. Procédé de fabrication d'une composition gazeuse inhalable selon l'une des revendications précédentes, contenant de l'oxygène en une proportion supérieure à 50% en volume, destinée à traiter, à minimiser ou à prévenir un état de migraine chronique chez un être humain, dans lequel ladite composition gazeuse est produite sur site, notamment au domicile d'un patient, par un dispositif de production d'oxygène conçu pour produire un flux de gaz contenant plus de 50% en volume d'oxygène à partir d'air, en particulier un concentrateur d'oxygène.

14. Méthode de traitement thérapeutique permettant de traiter, minimiser ou prévenir un état de migraine chronique chez un individu souffrant de migraines chroniques, dans laquelle :
i) on produit directement sur le lieu d'utilisation, notamment au domicile du patient, une composition gazeuse inhalable contenant plus de 50% d'oxygène, de préférence plus de 80% d'oxygène, au moyen d'un dispositif de production d'oxygène conçu pour produire un flux de gaz contenant plus de 50% d'oxygène à partir d'air ; et
ii) on administre par inhalation audit individu ladite composition gazeuse contenant de l'oxygène en une proportion supérieure à 50% en volume obtenu à l'étape i).
